# FASCICULE DE BREVET EUROPEEN

(11) **EP 3 860 353 B1**
(45) Date de publication et mention de la délivrance du brevet: **25.01.2023**
(21) Numéro de dépôt: 19801950.7
(22) Date de dépôt: 03.10.2019
(51) Int. Cl.: A23C 9/142, A23C 9/146, A23C 17/00, B01J 39/07, B01D 61/14

(54) **PROCEDE DE FABRICATION D'UN CONCENTRE DE PHOSPHOLIPIDES A PARTIR D'UNE COMPOSITION LAITIERE**
VERFAHREN ZUR HERSTELLUNG EINES PHOSPHOLIPIDKONZENTRATES AUS EINER MILCHZUSAMMENSETZUNG
METHOD FOR PRODUCING A PHOSPHOLIPID CONCENTRATE FROM A DAIRY COMPOSITION

(30) Priorité: 03.10.2018 FR 1859157
(43) Date de publication de la demande: 11.08.2021
(73) Titulaire: Euroserum, 70170 Port Sur Saone (FR)
(72) Inventeur: BASTIAN, Charlotte, 70000 Noidans-lès-Vesoul (FR); LAURENS, Lydie, 70400 Héricourt (FR); GIRARDOT, David, 25260 Montenois (FR)
(74) Mandataire: Delaveau, Sophie
(86) Numéro de dépôt international: PCT/FR2019/052344
(87) Numéro de publication internationale: WO 2020/070450

(56) Documents cités:
- EP-A1- 1 880 612
- EP-A1- 2 452 567
- WO-A1-02/34062
- WO-A1-2007/026053
- MORIN P ET AL: "Effect of Temperature and Pore Size on the Fractionation of Fresh and Reconstituted Buttermilk by Microfiltration", JOURNAL OF DAIRY SCIENCE, AMERICAN DAIRY SCIENCE ASSOCIATION, US, vol. 87, no. 2, 1 février 2004 (2004-02-01), pages 267-273, XP026979079, ISSN: 0022-0302 [extrait le 2004-02-01]
- BARRY K M ET AL: "Selective enrichment of dairy phospholipids in a buttermilk substrate through investigation of enzymatic hydrolysis of milk proteins in conjunction with ultrafiltration.", INTERNATIONAL DAIRY JOURNAL 2017 CORRESPONDENCE ADDRESS, P. M. KELLY, TEAGASC FOOD RESEARCH CENTRE, MOOREPARK, FERMOY, CO. CORK, REPUBLIC OF IRELAND. TEL. +353 87 7735352. E-MAIL PHIL.KELLY@TEAGASC.IE, vol. 68, 4 février 2017 (2017-02-04), pages 80-87, XP002790143,
- CORREDIG M ET AL: "Production of a Novel Ingredient from Buttermilk", JOURNAL OF DAIRY SCIENCE, AMERICAN DAIRY SCIENCE ASSOCIATION, US, vol. 86, 1 janvier 1998 (1998-01-01), pages 2744-2750, XP003018181, ISSN: 0022-0302, DOI: 10.3168/JDS.S0022-0302(03)73870-3
- SACHDEVA S ET AL: "RECOVERY OF PHOSPHOLIPIDS FROM BUTTERMILK USING MEMBRANE PROCESSING", KIELER WIRTSCHAFTLICHE FORSCHUNGSBERICHTE, VERLAG TH. MANN, GELSENKIRCHEN, DE, vol. 49, no. 1, 1 janvier 1997 (1997-01-01), pages 47-68, XP001014232, ISSN: 0023-1347
- KONRAD GERD ET AL: "Ultrafiltration of whey buttermilk to obtain a phospholipid concentrate", INTERNATIONAL DAIRY JOURNAL, ELSEVIER APPLIED SCIENCE, BARKING, GB, vol. 30, no. 1, 31 décembre 2012 (2012-12-31), pages 39-44, XP028968291, ISSN: 0958-6946, DOI: 10.1016/J.IDAIRYJ.2012.11.007
- SPENCE A J ET AL: "Phospholipid enrichment in sweet and whey cream buttermilk powders using supercritical fluid extraction", JOURNAL OF DAIRY SCIENCE, AMERICAN DAIRY SCIENCE ASSOCIATION, US, vol. 92, no. 6, 1 juin 2009 (2009-06-01), pages 2373-2381, XP026955427, ISSN: 0022-0302 [extrait le 2009-06-01]

## Description

### Domaine de l'invention

L'invention s'inscrit dans le domaine général des procédés de concentration de phospholipides contenus dans un produit laitier, et plus particulièrement de concentration de phospholipides à partir de babeurre.

### Art antérieur

Les phospholipides sont des constituants importants au niveau des membranes cellulaires. On les retrouve dans les organes vitaux tels que le cerveau, le foie et les reins. Ils interviennent dans de nombreux mécanismes biologiques tels que la régulation des réactions inflammatoires, la régulation du système immunitaire (lutte contre les infections chez les nourrissons, en particulier les prématurés), la lutte contre le cancer du côlon, la maturation intestinale du foetus, la baisse de la cholestérolémie, la prévention des maladies cardiovasculaires(rôle anti-athérogène), la réduction des allergies chez les jeunes enfants, l'aide à la détoxification du foie, l'amélioration du développement cognitif (particulièrement chez les nourrissons),la lutte contre Alzheimer ou encore le développement de la mémoire.

Les phospholipides sont naturellement présents dans le lait maternel. En industrie, on les retrouve ainsi dans différents produits et coproduits issus du lait d'origine bovine ou d'autre origine animale. Les phospholipides laitiers sont notamment contenus dans une fine membrane d'origine biologique appelée MFGM (Milk Fat Globule Membrane). La MFGM est une bicouche formée dans la glande mammaire, qui entoure les globules gras et permet de stabiliser l'émulsion de la matière grasse butyrique dans la phase aqueuse.

La MFGM est un mélange complexe de protéines et de lipides. La fraction lipidique de la MFGM issue de lait d'origine bovine est composée d'un mélange de 65% de lipides neutres (par exemple tri et di glycérides, cholestérol) et de 35% de lipides polaires ou complexes (dont 26% de phospholipides et 9% de sphingolipides). La famille des phospholipides se décompose de la façon suivante : 30% phosphatidylethanolamine (PE), 35% phosphatidylcholine (PC), 5% phosphatidylinositol (PI), 3% phosphatidylsérine (PS) et 22% sphingomyéline (SM) (Danthine et al., 2000).

Les phospholipides laitiers (d'origine animale) sont les phospholipides qui se rapprochent qualitativement le plus des phospholipides maternels, comparé notamment aux phospholipides d'origine végétale (soja notamment) qui ne contiennent pas de sphingomyéline.

On trouve notamment les phospholipides laitiers dans le babeurre, dit babeurre de lait, constituant la phase aqueuse qui résulte de la rupture de la MFGM lors du barattage de la crème et qui est récupérée lors de la formation des grains de beurre. En moyenne, la teneur en phospholipides du babeurre représente 20% de ses lipides totaux. Cette teneur varie selon l'origine de la crème, le type de procédé utilisé, mais aussi suivant le stade de lactation et la race de la vache. On trouve également des phospholipides dans le babeurre secondaire, babeurre de fonte ou babeurre de concentration (encore dénommé sérum de beurre), obtenu par concentration du beurre, et dans le béta-sérum obtenu directement à partir de la crème.

L'obtention de phospholipides au niveau industriel se fait généralement par des procédés d'extraction physique (broyage, filtration, centrifugation, fractionnement puis précipitation) et d'extraction par solvant (acétone en général).

On comprend qu'il existe un intérêt de concentrer les phospholipides contenus dans le babeurre, notamment pour l'exploitation des propriétés nutritionnelles et émulsifiantes du concentré.

La publication Microfiltration of Butter Sérum Upon Casein Micelle Destabilization, Rombaut et al, J.Dairy Sci.89 :1915-1925 décrit une tentative d'élaboration d'un concentré de phospholipides à partir d'un babeurre de concentration. Cette élaboration induit une concentration par microfiltration à 50°C sur une membrane en acétate de cellulose, de diamètre de pores de 0,15 µm et sous une pression transmembranaire de 1 Bar. Préalablement à la microfiltration, on cherche à dissocier les micelles de caséines présentes dans le babeurre. Ces micelles présentent en effet une taille comparable à celle des particules de MFGM, ce qui rend impossible la purification par microfiltration des phospholipides issus du babeurre. Pour ce faire, la dissociation est opérée par ajout de citrate de sodium ou d'éthanol au babeurre. Bien que la dissociation des micelles soit effective, notamment par ajout de citrate de sodium, il est observé un encrassement de la membrane et un taux de rétention insuffisant en phospholipides. La publication Effect of Température and Pore Size on the Fractionation of Fresh and Reconstituted Buttermilk by Microfiltration, Morin et al, J. Dairy Sci, 87:267-273 décrit également une concentration par microfiltration.

### Inconvénients de l'art antérieur

Outre un taux de rétention insuffisant en phospholipides, le procédé de la publication précitée implique l'utilisation de composés tels que le citrate de sodium et/ou l'éthanol qui sont non souhaités pour une utilisation du concentré dans le domaine nutritionnel, et plus particulièrement comme ingrédient de formule infantile.

### Objectifs de l'invention

L'invention vise principalement un procédé de concentration en phospholipides d'une composition laitière par procédé membranaire, assurant simultanément une rétention satisfaisante en phospholipides et une transmission suffisante en protéines, étant entendu que ce procédé doit être exempt de tout composé incompatible et/ou non souhaité avec les applications visées, notamment dans le domaine nutritionnel.

### Exposé de l'invention

A cet effet, le procédé de l'invention de fabrication d'un concentré de phospholipides issus d'une composition laitière liquide comprenant au moins des caséines et des phospholipides, est essentiellement caractérisé en ce qu'il comprend au moins les étapes de :
- passage de la composition laitière dans une colonne à échange d'ions sur résine cationique,
- concentration des phospholipides de la composition laitière appauvrie en calcium par microfiltration à gradient de pression transmembranaire contrôlé, et
- récupération du rétentat de la microfiltration et obtention d'un concentré de phospholipides.

Le procédé de l'invention peut également comporter les caractéristiques optionnelles suivantes considérées isolément ou selon toutes les combinaisons techniques possibles :
- la composition laitière comprend du babeurre de lait et/ou du babeurre de concentration.
- le rapport entre le facteur de concentration en protéines et le facteur de concentration en phospholipides est inférieur à 1, chacun desdits facteurs de concentration correspondant au rapport entre la fraction massique sur l'extrait sec dans le rétentat et la fraction massique sur l'extrait sec dans le babeurre avant microfiltration, et en ce que le taux de rétention en protéines induit par la microfiltration est inférieur à 70%.
- le rapport entre le facteur de concentration en protéines et le facteur de concentration en phospholipides est inférieur à 0,9.
- la résine cationique de la colonne à échange d'ions est une résine cationique faible.
- une diafiltration est réalisée au cours de l'étape de concentration des phospholipides.
- la diafiltration est appliquée pour un facteur de concentration volumique inférieur à 3.
- l'étape de concentration des phospholipides est réalisée à 50°C.
- la vitesse tangentielle appliquée à la microfiltration est supérieure à 4 m/s.
- la taille de pores moyenne de la membrane de microfiltration est inférieure à 0,8 micromètres.
- la taille de pores moyenne de la membrane de microfiltration est inférieure à 0,5 micromètres.
- la pression transmembranaire est comprise entre 0,4 Bar et 2 Bar.
- la pression transmembranaire est comprise entre 0,6 Bar inclus et 1 Bar inclus.
- selon une première variante, l'étape de concentration des phospholipides est réalisée sur une membrane à gradient de perméabilité.
   ∘ la membrane à gradient de perméabilité présente une taille moyenne de pores de 0,1 micromètres.
   ∘ la pression transmembranaire est maintenue à 1 Bar.
   ∘ une diafiltration continue est appliquée lorsque le facteur de concentration volumique est compris entre 1,2 et 3.
   ∘ l'étape de concentration est réalisée jusqu'à l'obtention d'un facteur de concentration volumique de la composition laitière compris entre 2,5 et 5.
- selon une seconde variante, l'étape de concentration des phospholipides est réalisée sur une membrane céramique, couplée à un système d'uniformisation de la pression transmembranaire.
   ∘ la taille moyenne des pores est comprise entre 0,1 et 0,2 micromètres inclus.
   ∘ la pression transmembranaire est maintenue à 0,6 Bar.
   ∘ une diafiltration continue est appliquée lorsque le facteur de concentration volumique est compris entre 1,2 et 4.
   ∘ l'étape de concentration est réalisée jusqu'à l'obtention d'un facteur de concentration volumique de la composition laitière compris entre 2,5 et 5.

### Présentation des figures

D'autres caractéristiques et avantages de l'invention ressortiront clairement de la description qui en est donnée ci-dessous, à titre indicatif et nullement limitatif, en référence aux figures suivantes :
- La figure 1 illustre les taux de rétention en protéines en fonction de la pression transmembranaire appliquée dans un procédé de concentration membranaire couplé à un système d'uniformisation de la pression transmembranaire, précédé ou non d'une étape de déminéralisation,
- La figure 2 illustre la quantité de Matière Azotée Totale retrouvée dans un culot de centrifugation à 31500 rpm par rapport au pourcentage de Matière Azotée Initiale d'un concentré obtenu selon le procédé de l'invention, et un concentré obtenu sans déminéralisation,
- La figure 3 illustre le poids en eau par rapport au poids en Matière Azotée Totale dans le même culot de centrifugation d'un concentré obtenu selon le procédé de l'invention, et un concentré obtenu sans déminéralisation,
- La figure 4 illustre le spectre granulométrique d'un concentré obtenu selon le procédé de l'invention, et d'un concentré obtenu sans déminéralisation, en présence d'EDTA, et
- La figure 5 illustre le spectre granulométrique d'un concentré obtenu selon le procédé de l'invention, et d'un concentré obtenu sans déminéralisation, en présence d'EDTA et de SDS.

### Description détaillée de l'invention

Le procédé de fabrication d'un concentré de phospholipides de l'invention à partir d'une composition laitière contenant des caséines et des phospholipides comporte deux étapes principales : la première est une étape de dissociation des caséines et la seconde une étape de concentration par microfiltration à gradient de pression transmembranaire contrôlée.

La matière première utilisée est du babeurre de lait ou du babeurre de concentration en raison de la forte teneur en phospholipides (environ 20%) dans ses lipides. On pourra utiliser alternativement un mélange de babeurre de lait et de babeurre de concentration. Le procédé de l'invention s'applique également à toute autre type de babeurre, et de façon plus générale à toute composition laitière comportant des caséines et des phospholipides.

La caséine qui est présente dans les compositions laitières et en particulier dans le babeurre est sous forme micellaire, et présente une taille similaire aux fragments de MFGM (Milk Fat Globule Membrane) qui contiennent les phospholipides, les autres composés présents dans le babeurre étant taille différente. Il s'ensuit que le passage sur membrane du babeurre comportant de la caséine sous forme micellaire conduirait à retenir à la fois les protéines et les phospholipides, en ne permettant pas de concentrer préférentiellement les phospholipides.

Il s'agit donc de réduire la taille des micelles de caséines pour diminuer le taux de rétention des protéines sur membrane. Pour ce faire et comme décrit dans la publication susmentionnée, il est connu de chélater le calcium des micelles de caséine par l'ajout d'un agent dissociant tel que du citrate de sodium ou de l'éthanol. Mais outre l'impossibilité décrite dans cette publication de réaliser le concentré visé, de telles méthodes utilisant des solvants entraînent une perte des qualités nutritionnelles du concentré obtenu.

Selon l'invention, la diminution de la taille des micelles de caséines est obtenue par élimination du calcium par échange d'ions sur une résine cationique. De cette façon, les micelles de caséines se dissocient pour former des particules de plus petites tailles que les fragments lipidiques contenant les phospholipides.

La résine cationique utilisée dans la colonne de déminéralisation est une résine faible dont l'affinité par rapport aux ions divalents est supérieure aux ions monovalents en permettant ainsi d'échanger majoritairement le calcium. La résine est régénérée sous forme de cation monovalent par exemple sous forme potassium, sodium ou hydrogène. On préfère la régénération sous forme sodium et potassium, permettant ainsi d'éviter l'acidification du babeurre (et ainsi l'ajout d'une base en sortie de colonne) et l'agrégation des caséines lors d'une régénération sous forme hydrogène. Le babeurre en sortie de la colonne de déminéralisation présente donc des submicelles de caséines libres et une teneur en calcium diminuée. Ce babeurre sera dénommé « babeurre ou composition laitière appauvri(e) en calcium dans la suite de la description ».

Selon l'invention, le babeurre appauvri en calcium en sortie de colonne est ensuite soumis à une microfiltration à gradient de pression transmembranaire contrôlé duquel est récupéré le rétentat qui forme le concentré de phospholipides. L'application d'une microfiltration à gradient de pression transmembranaire contrôlée est réalisée dans le cadre de l'invention pour les raisons suivantes. Lors d'une filtration membranaire, les performances de séparation sont améliorées d'une part en augmentant la vitesse tangentielle du produit le long de la membrane et d'autre part en maitrisant la pression transmembranaire. L'augmentation de la vitesse tangentielle a pour conséquence une perte de charge importante entre l'entrée et la sortie du produit et engendre ainsi la formation d'un gradient de pression transmembranaire le long de la membrane. La vitesse tangentielle rend donc difficile la maitrise de la pression transmembranaire. Des technologies ont donc été mises au point afin de palier à cette problématique. La première technologie connue est le système UTP c'est-à-dire l'ajout d'une recirculation au niveau du perméat afin de compenser la pression coté perméat par rapport à la pression subie coté rétentat. La deuxième technologie connue est les membranes dites GP à gradient de perméabilité qui ont la caractéristique de posséder un gradient de perméabilité le long de la membrane au niveau du support de filtration afin d'atténuer le gradient de pression transmembranaire. D'autres membranes existent avec le même système de gradient de perméabilité, que ce soit par des modifications de structure du support ou de la couche de filtration. Ces technologies membranaire sont qualifiées dans la suite de la description par « microfiltration à gradient de pression transmembranaire contrôlé ».

La concentration peut ainsi être réalisée sur une membrane à gradient de perméabilité (GP), par exemple commercialisée sous le nom Membralox^{®} GP, ou sur une membrane céramique couplée à un système d'uniformisation de la pression transmembranaire également dénommé système UTP (Uniform Transmembrane Pressure). On préfère l'utilisation d'une membrane à gradient de pression pour la simplicité des installations. D'autres technologies équivalentes peuvent être utilisées telles que les membranes tubulaires céramiques commercialisées sous le nom ISOFLUX^{™}.

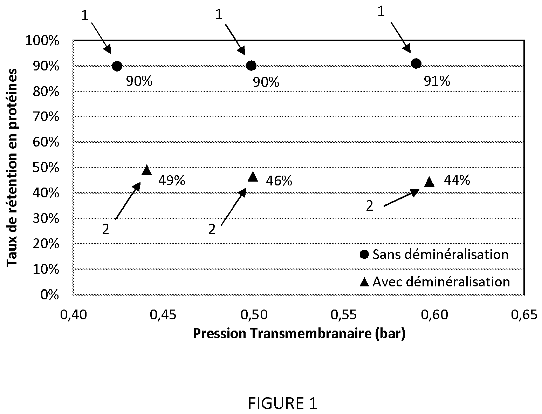

On se réfère aux Tableaux 1a et 1b ci-dessous, ainsi qu'à la figure 1 pour décrire les essais réalisés et constater l'efficacité de la concentration des phospholipides par le procédé de l'invention.

Pour les essais réalisés avec le système UTP, le babeurre est frais, ou préparé à 8% massique d'extrait sec à partir de poudre de babeurre. Les essais se déroulent en batch avec recirculation du rétentat et soutirage continu du perméat. Des échantillons de retentât et perméat sont prélevés simultanément. Pour assurer le meilleur compromis entre la rétention des phospholipides et la transmission des protéines, la porosité de la membrane céramique est de 0,2 µm. On pourra alternativement utiliser une porosité de 0,1 µm. La vitesse tangentielle élevée supérieure à 4m/s, de préférence de 7m/s, est choisie pour améliorer la transmission des protéines. Des températures opératoires de 30°C et 50°C et différentes pressions transmembranaires entre 0,4 et 0,6 bar sont testées. La concentration en batch est réalisée de la façon suivante : on opère en premier lieu à une concentration volumique comprise entre 1,2 et 4 de préférence par 2 du babeurre, puis on réalise une diafiltration instantanée par ajout d'eau à hauteur de 50% du volume initial en babeurre, et on poursuit la concentration jusqu'à un facteur de concentration volumique compris entre 2,5 et 5, de préférence par 3.

Les applications du concentré obtenu par le procédé de l'invention sont multiples. En premier lieu, le concentré peut être utilisé comme ingrédient sous forme liquide, concentrée ou pulvérulente à des fins nutritionnelles. On citera à titre d'exemple la nutrition infantile, senior et clinique, ou encore la nutrition adulte ciblée, pour les femmes enceintes et les sportifs par exemple. Dans le cadre des formules infantiles, le concentré constitue un ingrédient et permet ainsi la réalisation d'un lait infantile comportant un tel concentré.

Le concentré peut trouver d'autres applications eu égard à ses propriétés émulsifiantes. On citera à titre non limitatif des applications pour la préparation de crèmes glacées, produits de BVP (biscuiterie/viennoiserie/pâtisserie), chocolaterie et confiserie, ou encore fromages fondus.

La figure 1 illustre les taux de rétention en protéines obtenus lors d'essais réalisés avec (références 2) et sans (références 1) déminéralisation préalable, et avec le système UTP tel que précédemment décrit à 50°C pour des pressions transmembranaires variables. On constate une forte efficacité du procédé de l'invention, et plus particulièrement de l'effet de la déminéralisation, sur le taux de rétention des protéines qui passe de 90% environ sans déminéralisation à moins de 50% avec déminéralisation. On constate également une sensible influence de la pression transmembranaire sur le taux de transmission de protéines lorsqu'une déminéralisation préalable est réalisée, c'est pourquoi une pression transmembranaire de 0,6 bar est préférée.

Pour les essais réalisés avec une membrane GP, le babeurre est également préparé à 8% massique d'extrait sec à partir de poudre de babeurre. La porosité de la membrane est de 0,1 µm, la vitesse tangentielle est de 5,5m/s, la température opératoire est de 50°C et la pression transmembranaire est de 1 bar. La concentration est réalisée de la façon suivante : on opère en premier lieu à une concentration volumique entre 1,2 et 3 du babeurre, de préférence de 1,5, puis on réalise une diafiltration en continu avec un débit identique au débit de perméat et une quantité d'eau de 100% en volume de babeurre initial, et on poursuit la concentration jusqu'à l'obtention d'un facteur de concentration compris entre 2,5 et 5, de préférence de 4. Les paramètres opératoires testés sont indiqués dans le Tableau 1a ci-dessous.

Que ce soit pour les essais réalisés avec le système UTP ou avec la membrane GP, la diafiltration, technique connue pour améliorer la concentration d'un produit par filtration, est très préférentiellement mise en oeuvre pour atteindre le taux de phospholipides souhaité. La diafiltration peut être continue ou instantanée pour les deux types de membranes. On applique de préférence la diafiltration à un facteur de concentration faible, de préférence inférieur à 2, pour assurer au mieux la transmission des protéines à travers la membrane. On pourrait alternativement opérer la diafiltration dès le début de l'étape de concentration.

Pour ce qui est de la porosité de la membrane, une porosité de 0,8 µm n'est pas suffisamment discriminante entre les protéines et les phospholipides, ce dont il résulte une concentration insuffisante en phospholipides. La porosité de la membrane est donc choisie comme étant inférieure à 0,8 µm, et de préférence inférieure à 0,5 µm pour assurer une efficacité de rendement optimale.

La vitesse tangentielle de la microfiltration opérée avec le système UTP et la membrane GP est préférentiellement élevée, soit supérieure à 4 m/s pour améliorer la transmission des protéines à travers la membrane. Et la température de 50°C est préférentiellement choisie pour augmenter le débit de perméat sans changer les capacités de séparation du procédé.

On se réfère au Tableau 1b qui présente les résultats des essais menés sur membrane organique, sur membrane GP et avec le système UTP selon les paramètres opératoires définis précédemment. Pour tous les essais, le facteur de concentration volumique appliqué est de 3. Les indications soulignées correspondent à des essais dont les paramètres de procédé se situent en dehors de l'invention, et les indications non soulignées correspondent à l'inverse à des essais dont les paramètres de procédé s'inscrivent dans le cadre de l'invention. Le facteur de concentration (FC) indiqués dans le Tableau 1a permet de rendre compte des taux de rétention en protéines et en phospholipides en s'affranchissant de la variabilité de la teneur en extrait sec du babeurre destiné à être concentré.

Le concentré en phospholipides obtenu par le procédé de l'invention présente un rapport entre sa teneur en phospholipides sur l'extrait sec et sa teneur en protéines sur l'extrait sec qui est supérieur à 6,5. Avantageusement, ce rapport est supérieur à 7.

| N° Essai | Type de membrane | Avec ou sans déminéralisation | Babeurre | Seuil de coupure (µm) | T | Diafiltration | PTM |
|---|---|---|---|---|---|---|---|
| 1 | Membrane organique | Sans | Reconstitué | 0,1 | 50 °C | Continue | 1 Bar |
| 2 | | Avec | Reconstitué | 0,1 | 50 °C | Continue | 1 Bar |
| 3 | Membrane GP | Sans | Reconstitué | 0,1 | 50°C | Continue | 1 bar |
| 4 | | Avec | Reconstitué | 0,1 | 50°C | Continue | 1 bar |
| 5 | | Avec | Reconstitué | 0,1 | 50 °C | Continue | 1 Bar |
| 6 | | Avec | Frais | 0,1 | 50 °C | Continue | 1 Bar |
| 7 | | Avec | Frais | 0,1 | 50 °C | Continue | 1 Bar |
| 8 | Système UTP | Sans | Reconstitué | 0,2 | 50 °C | Instantanée | 0,6 Bar |
| 9 | | Sans | Frais | 0,2 | 50 °C | Instantanée | 0,6 Bar |
| 10 | | Avec | Reconstitué | 0,2 | 50 °C | Instantanée | 0,6 Bar |
| 11 | | Avec | Frais | 0,2 | 50 °C | Instantanée | 0,6 Bar |
| 12 | | Sans | Reconstitué | 0,2 | 30 °C | Instantanée | 0,6 Bar |
| 13 | | Avec | Reconstitué | 0,2 | 30 °C | Instantanée | 0,6 Bar |
| 14 | | Avec | Reconstitué | 0,2 | 30 °C | Instantanée | 0,6 Bar |
| 15 | | Sans | Reconstitué | 0,2 | 50 °C | Instantanée | 0,4 Bar |
| 16 | | Avec | Reconstitué | 0,2 | 50 °C | Instantanée | 0,4 Bar |
| 17 | | Avec | Reconstitué | 0,2 | 50 °C | Instantanée | 0,4 Bar |
| 18 | | Sans | Reconstitué | 0,2 | 50 °C | Instantanée | 0,6 Bar |
| 19 | | Avec | Reconstitué | 0,2 | 50 °C | Instantanée | 0,6 Bar |
| 20 | | Avec | Reconstitué | 0,2 | 50 °C | Instantanée | 0,6 Bar |
| 21 | | Avec | Frais | 0,2 | 50 °C | Instantanée | 0,6 Bar |

| | | | | | | | |
|---|---|---|---|---|---|---|---|
| Tableau 1a : Paramètres opératoires des essais menés sur membrane organique, sur membrane GP et avec le système UTP | | | | | | | |

**Tableau 1b : Résultats comparatifs des essais menés sur membrane organique, sur membrane GP et avec le système UTP**

| N° Essai | Rétention* extrait sec (%) | Rétention* protéines (%) | Rétention* Phospholipides (%) | FC** protéines | FC** phospholipides | FC** protéines/FC phospholipides | Teneur en phospholipides (%ES) dans le concentré | Teneur en protéines (%ES) dans le concentré | Phospholipides (%ES)/Protéines (%ES) dans le concentré |
|---|---|---|---|---|---|---|---|---|---|
| 1 | 41 | 86 | 90 | 2,2 | 2,2 | 1,0 | 4,2 | 69 | 6,0% |
| 2 | 38 | 84 | 87 | 2,1 | 2,3 | 0,9 | 4,3 | 73 | 5,9% |
| 3 | 41 | 85 | 74 | 2,1 | 1,8 | 1,2 | 3,3 | 74 | 4,5% |
| 4 | 32 | 67 | 83 | 2,1 | 2,6 | 0,8 | 4,8 | 71 | 6,7% |
| 5 | 29 | 57 | 87 | 2 | 3,0 | 0,7 | 5,6 | 72 | 7,8% |
| 6 | 37 | 66 | 75 | 1,8 | 2,0 | 0,9 | 4,5 | 63 | 7,2% |
| 7 | 20 | 38 | 80 | 1,9 | 3,9 | 0,5 | 8 | 67 | 11,9% |
| 8 | 44 | 88 | ND | 2 | ND | ND | ND | 64 | ND |
| 9 | 46 | 89 | ND | 2 | ND | ND | ND | 69 | ND |
| 10 | 26 | 43 | 67 | 1,7 | 2,6 | 0,7 | 4,8 | 55 | 8,7% |
| 11 | 27 | 38 | 71 | 1,4 | 2,6 | 0,5 | 5,3 | 49 | 10,9% |
| 12 | 39 | 78 | 74 | 2 | 1,9 | 1,1 | 3,5 | 65 | 5,4% |
| 13 | 31 | 46 | 76 | 1,5 | 2,5 | 0,6 | 4,7 | 52 | 9,0% |
| 14 | 28 | 45 | 74 | 1,6 | 2,6 | 0,6 | 4,8 | 53 | 9,1% |
| 15 | 42 | 88 | 97 | 2,1 | 2,3 | 0,9 | 4,5 | 73 | 6,2% |
| 16 | 29 | 43 | 101 | 1,5 | 3,6 | 0,4 | 5,9 | 54 | 10,9% |
| 17 | 27 | 40 | 68 | 1,5 | 2,6 | 0,6 | 4,7 | 50 | 9,4% |
| 18 | 44 | 88 | 102 | 2 | 2,3 | 0,9 | 3,8 | 64 | 5,9% |
| 19 | 21 | 37 | 81 | 1,8 | 3,3 | 0,5 | 6,2 | 56 | 11,1% |
| 20 | 25 | 43 | 67 | 1,7 | 2,6 | 0,7 | 4,8 | 55 | 8,7% |
| 21 | 33 | 38 | 71 | 1,4 | 2,6 | 0,5 | 5,3 | 48,9 | 10,8% |

| | | | | | | | | | |
|---|---|---|---|---|---|---|---|---|---|
| *Taux de rétention : rapport entre la masse dans le rétentat et la masse dans le babeurre avant microfiltration ; **FC - Facteur de Concentration : rapport entre la fraction massique sur l'extrait sec dans le rétentat et la fraction massique sur l'extrait dans le babeurre avant microfiltration | | | | | | | | | |

On constate en premier lieu que lorsque la microfiltration est conduite sur membrane organique (essais N°1 et 2), le taux de rétention des protéines reste élevé à plus de 80% y compris lorsque le babeurre a été préalablement appauvri en calcium. Il n'y a donc aucun effet de la déminéralisation sur la transmission des protéines alors même que l'inverse était attendu. Il réside également une difficulté de nettoyage de ce type de membrane après la concentration du babeurre.

Concernant les essais réalisés sur membrane GP, on constate que le taux de rétention en protéines est significativement abaissé lorsque le babeurre est préalablement appauvri en calcium. Si le facteur de concentration en protéines n'est pas significativement abaissé, le facteur de concentration en phospholipides est fortement augmenté ce qui permet d'obtenir d'une part un rapport entre le facteur de concentration en protéines et le facteur de concentration en phospholipides qui est abaissé par rapport à celui obtenu sans déminéralisation et un rapport entre phospholipides et protéines dans le concentré bien supérieur à celui obtenu sans déminéralisation. On constate également que les résultats sont comparables entre le babeurre reconstitué et le babeurre frais.

Concernant les essais réalisés avec le système UTP, on constate également que le taux de rétention en protéines est significativement abaissé lorsque le babeurre est préalablement appauvri en calcium pour un taux de rétention en phospholipides élevé. Le facteur de concentration en protéines est abaissé par rapport à des essais menés sans déminéralisation, ce qui engendre une baisse substantielle du rapport entre le facteur de concentration en protéines et le facteur de concentration en phospholipides. Le rapport entre phospholipides et protéines dans le concentré est par ailleurs très supérieur à celui obtenu sans déminéralisation. On constate, comme pour les essais réalisés avec la membrane GP, que les résultats sont comparables entre le babeurre reconstitué et le babeurre frais.

Partant de ces résultats, on définit deux paramètres conjoints caractéristiques du procédé de l'invention. Il s'agit du rapport entre le facteur de concentration en protéines et le facteur de concentration en phospholipides qui est inférieur à 1, et du taux de rétention en protéines qui est inférieur à 70%. Préférentiellement, pour assurer une meilleure concentration en phospholipides dans le concentré, le rapport entre le facteur de concentration en protéines et le facteur de concentration en phospholipides est inférieur à 0,9.

On décrit ci-après deux exemples (Exemples 1 et 2) de réalisation non limitatifs de l'invention et un exemple de caractérisation des composés lipidiques et protéiques dans le concentré de l'invention et dans un concentré dont l'élaboration est dénué de déminéralisation préalable (Exemple 3).

### Exemple 1

### Préparation de la résine :

La résine utilisée est une résine échangeuse d'ions cationique faible, elle est constituée de groupements carboxyliques sur une matrice acrylique (IMAC HP336). La résine est à l'origine sous forme hydrogène H+. Une régénération avec une solution d'hydroxyde de sodium NaOH et d'hydroxyde de potassium KOH est appliquée pour convertir 20 litres de résines neuves sous forme sodium et potassium. Pour cela, une solution équimolaire de 75L est préparée à 0,5mol/L de NaOH et 0,5 mol/L de KOH. La solution est éluée sur le lit de résine à un débit de 6 BV (volume du lit de résines)/h. La résine est ensuite rincée à l'eau au même débit jusqu'à l'obtention d'une conductivité de l'eau en sortie du lit inférieure à 1mS/cm.

### Passage du babeurre sur la résine :

Le babeurre de lait issu de la fabrication du beurre a été préalablement traité par bactofugation et pasteurisation. Sa composition est donnée dans le tableau ci-dessous. Un volume de 40 BV de babeurre est élué sur la résine à un débit volumique de 10 BV/h. Une pousse du produit est ensuite effectuée avec 1BV d'eau.

**Tableau 1 : Composition du babeurre avant et après traitement sur la colonne échangeuse d'ions**

| Composition du babeurre | | Avant l'échange d'ions | Après l'échange d'ions |
|---|---|---|---|
| **Extrait sec** | % | 7,8 | 7,8 |

| **Composition massique sur l'extrait sec** | | | |
|---|---|---|---|
| **Protéines (=N×6,38)** | % | 35,9 | 35,3 |
| **Na** | mg/100g | 477 | 1202 |
| **K** | mg/100g | 1711 | 3019 |
| **Ca** | mg/100g | 1102 | 4 |
| **Mg** | mg/100g | 119 | 1 |
| **Cendres** | % | 8,0 | 9,5 |

La résine est ensuite régénérée à l'acide chlorhydrique de concentration 1,7 mol/L et de volume 46 L. Le débit de percolation de la solution appliqué est de 6 BV/h. Puis la résine est rincée avec de l'eau jusqu'à atteindre une conductivité en sortie de colonne de 1 mS/cm. La résine est ensuite régénérée sous forme Na/K avec le même protocole de préparation que précédemment.

### Concentration par microfiltration :

Le babeurre traité sur la colonne échangeuse d'ions est chauffé à 50°C avant d'être concentré sur la microfiltration. Le système de microfiltration utilisé est un module de 19 membranes Membralox GP EP1940 de seuil de coupure de 0,1µm. Le babeurre est concentré en batch, c'est-à-dire avec recirculation du rétentat dans un bac jusqu'à atteinte du facteur de concentration volumique souhaité. Dès que le facteur de concentration volumique est de 1,5, une diafiltration continue est appliquée. Pour cela, l'eau est ajoutée dans le bac à un débit continu de 160L/h, le volume total d'eau ajouté est égal à 80% du volume de babeurre initial. L'eau de diafiltration est de même à 50°C et la température dans le procédé est maintenue à 50°C grâce à un échangeur thermique. Le pilotage de la filtration est réalisé à pression transmembranaire constante de 1 bar tout au long de la concentration. Le perméat est récupéré dans un bac. La concentration est terminée lorsque le facteur de concentration volumique FCV est de 4 par rapport au volume initial de babeurre.

La composition du babeurre en début et en fin de concentration est indiquée dans le tableau suivant.

**Tableau 2 : Composition du babeurre avant concentration et après appauvrissement en calcium, et après concentration**

| **Composition du babeurre** | | **Initial** | **Concentré** |
|---|---|---|---|
| **Extrait sec** | **%** | 7,8 | 6,7 |
| **Protéines** | **% ES** | 35,3 | 67,3 |
| **Phospholipides** | **% ES** | 2,0 | 8,0 |

### Exemple 2

Après une première étape d'échange d'ions identique à celle décrite pour l'exemple 1, le babeurre appauvri en calcium est concentré en batch sur une membrane de microfiltration céramique de 0,2 µm. Le module est composé de 7 membranes Membralox P3730. Le procédé est muni d'un système UTP (Uniform Transmembrane Pressure) c'est-à-dire qu'il est composé d'une pompe de recirculation coté perméat. La pompe de recirculation fonctionne à un débit de 42 m3/h afin d'atteindre une vitesse de 7m/s dans le module de filtration. La concentration est effectuée à une pression transmembranaire maintenue constante à 0,6 Bar tout au long de l'étape du procédé. Comme pour l'exemple 1, le babeurre est tout d'abord chauffé à 50°C et maintenu à cette température grâce à un échangeur thermique.

Le babeurre est concentré jusqu'à un FCV de 2, puis une diafiltration instantanée est appliquée en ajoutant un même volume d'eau que de produit concentré dans le bac. La concentration est ensuite poursuivie jusqu'à un FCV de 3.

Les compositions du babeurre initial et du babeurre concentré sont données dans le tableau suivant.

**Tableau 3 : Composition du babeurre avant et après concentration**

| **Composition du babeurre** | | **Initial** | **Concentré** |
|---|---|---|---|
| **Extrait sec** | **%** | 7,8 | 6,7 |
| **Protéines** | **% ES** | 35,3 | 67,3 |
| **Phospholipides** | **% ES** | 2,0 | 8,0 |

### Exemple 3

Des tests de caractérisation ont été menés sur le concentré en phospholipides liquide de l'invention tel qu'obtenu à l'Exemple 1 et sur un concentré liquide préparé selon l'Exemple 1 mais dont le procédé de fabrication est dénué de l'étape de déminéralisation.

Le premier test mené consiste à réaliser une centrifugation à 31500 rpm sur les deux concentrés. Cette vitesse de centrifugation correspond, selon la littérature, à la vitesse assurant la précipitation des micelles de caséines.

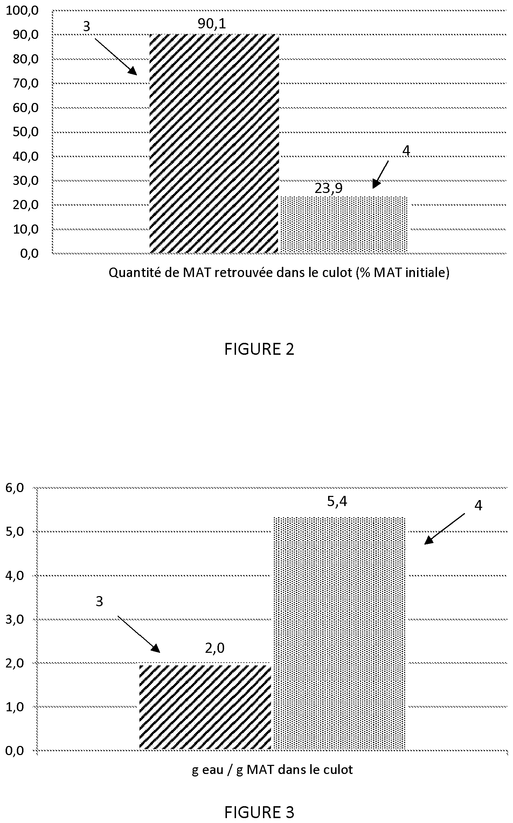

La figure 2 illustre la quantité de matière azotée totale (MAT) retrouvée dans le culot de centrifugation par rapport au pourcentage de matière azotée totale initiale pour, respectivement, le concentré obtenu sans déminéralisation (référence 3) et le concentré de l'invention (référence 4). On constate une présence substantiellement moins importante matière azotée dans le concentré de l'invention. L'identification des micelles de caséines dans le concentré obtenu sans déminéralisation (et leur quasi absence dans le concentré de l'invention) est confortée par les résultats reportés sur la figure 3. La figure 3 illustre le poids, exprimé en gramme, d'eau par rapport au poids, exprimé en gramme, de matière azotée totale (MAT) dans le culot de centrifugation pour, respectivement, le concentré obtenu sans déminéralisation (référence 3), et le concentré de l'invention (référence 4). Pour le concentré obtenu sans déminéralisation, la rétention d'eau d'une valeur de 2 grammes d'eau par gramme de Matière Azotée Totale obtenue est conforme à celle connue de la littérature pour les micelles de caséines. La valeur plus élevée de 5,4 grammes d'eau par gramme de Matière Azotée Totale pour le concentré de l'invention indique que la matière azotée totale présente dans ce concentré correspond à la présence de fragments de micelles, et non de micelles de caséines. Le concentré de l'invention ne contient ainsi pas de micelles de caséines, contrairement au concentré obtenu sans déminéralisation.

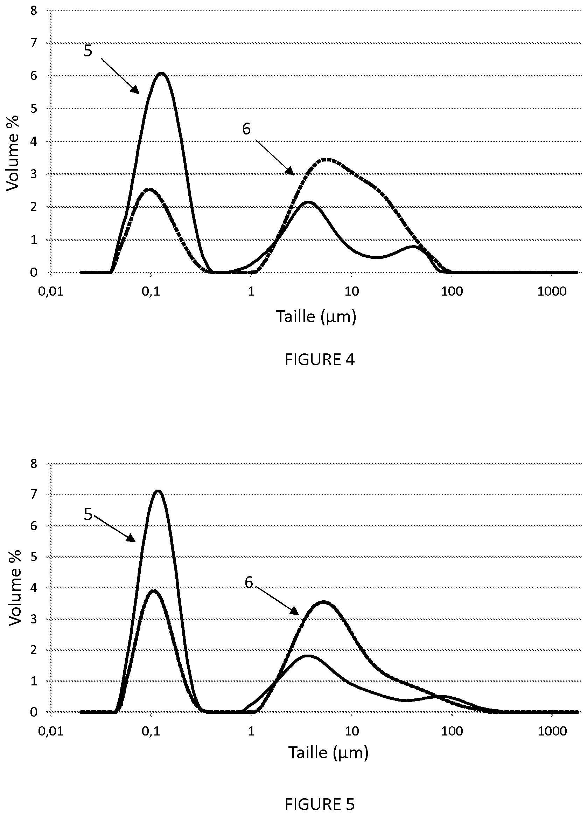

Des mesures de granulométrie laser sont réalisées sur le concentré liquide de l'invention (référence 6) et sur le concentré obtenu sans déminéralisation (référence 5). La figure 4 illustre les résultats obtenus lorsque de l'EDTA (acide éthylènediaminetétraacétique) est ajouté aux concentrés, et la figure 5 illustre les résultats obtenus lorsque de l'EDTA et du SDS (dodécylsulfate de sodium) sont ajoutés aux concentrés. L'EDTA est un chélateur de calcium et permet de désagréger les micelles de caséines. Le SDS désagrège les globules gras les uns des autres.

On constate sur les deux figures 4 et 5 une présence nettement plus importante des caséines (aires sous les courbes respectives correspondant aux pics centrés sur 0,1 µm) pour le concentré obtenu sans déminéralisation, montrant une fois encore la présence importante de micelles de caséines lorsque la déminéralisation sur colonne échangeuse d'ions n'est pas appliquée.

On constate également, en présence de SDS, pour le concentré de l'invention, une atténuation de l'aire sous la courbe positionnée entre 10 et 100 µm et une augmentation concomitante de l'aire sous la courbe positionnée entre 1 et 10 µm, alors que pour le concentré obtenu sans déminéralisation ces aires n'évoluent quasiment pas en présence de SDS. Ces résultats montrent la présence, dans le concentré de l'invention, des agglomérations de membranes de globules gras (de taille comprise entre 10 et 100 µm) qui se désagrègent en présence de SDS. La présence de membranes de globules gras indiquant la présence de phospholipides, ces résultats indiquent et confortent la concentration substantielle en phospholipides obtenue grâce au procédé de l'invention.

## Revendications

1. Procédé de fabrication d'un concentré de phospholipides issus d'une composition laitière liquide comprenant au moins des caséines et des phospholipides, **caractérisé en ce qu'**il comprend au moins les étapes de :
- passage de la composition laitière dans une colonne à échange d'ions sur résine cationique,
- concentration des phospholipides de la composition laitière appauvrie en calcium par microfiltration à gradient de pression transmembranaire contrôlé, et
- récupération du rétentat de la microfiltration et obtention d'un concentré de phospholipides.

2. Procédé selon la revendication précédente, **caractérisé en ce que** la composition laitière comprend du babeurre de lait et/ou du babeurre de concentration.

3. Procédé selon l'une quelconque des revendications précédentes, **caractérisé en ce que** le rapport entre le facteur de concentration en protéines et le facteur de concentration en phospholipides est inférieur à 1, chacun desdits facteurs de concentration correspondant au rapport entre la fraction massique sur l'extrait sec dans le rétentat et la fraction massique sur l'extrait sec dans le babeurre avant microfiltration, et **en ce que** le taux de rétention en protéines induit par la microfiltration est inférieur à 70% et **en ce que** le rapport entre le facteur de concentration en protéines et le facteur de concentration en phospholipides est inférieur à 0,9.

4. Procédé selon l'une quelconque des revendications précédentes, **caractérisé en ce que** la résine cationique de la colonne à échange d'ions est une résine cationique faible.

5. Procédé selon l'une quelconque des revendications précédentes, **caractérisé en ce qu'**une diafiltration est réalisée au cours de l'étape de concentration des phospholipides.

6. Procédé selon la revendication précédente, **caractérisé en ce que** la diafiltration est appliquée pour un facteur de concentration volumique inférieur à 3.

7. Procédé selon l'une quelconque des revendications précédentes, **caractérisé en ce que** l'étape de concentration des phospholipides est réalisée à 50°C.

8. Procédé selon l'une quelconque des revendications précédentes, **caractérisé en ce que** la vitesse tangentielle appliquée à la microfiltration est supérieure à 4 m/s.

9. Procédé selon l'une quelconque des revendications précédentes, **caractérisé en ce que** la taille de pores moyenne de la membrane de microfiltration est inférieure à 0,8 micromètres, de préférence inférieure à 0,5 micromètres.

10. Procédé selon la revendication précédente, **caractérisé en ce que** la pression transmembranaire est comprise entre 0,4 Bar et 2 Bar, de préférence entre 0,6 Bar inclus et 1 Bar inclus.

11. Procédé selon l'une quelconque des revendications précédentes, **caractérisé en ce que** l'étape de concentration des phospholipides est réalisée sur une membrane à gradient de perméabilité.

12. Procédé selon la revendication précédente, **caractérisé en ce que** la membrane à gradient de perméabilité présente une taille moyenne de pores de 0,1 micromètres, et **en ce que** la pression transmembranaire est maintenue à 1 Bar.

13. Procédé selon l'une quelconque des revendications 11 et 12, **caractérisée en ce qu'**une diafiltration continue est appliquée lorsque le facteur de concentration volumique est compris entre 1,2 et 3, et de préférence **en ce que** l'étape de concentration est réalisée jusqu'à l'obtention d'un facteur de concentration volumique de la composition laitière compris entre 2,5 et 5.

14. Procédé selon l'une quelconque des revendications 1 à 10, **caractérisé en ce que** l'étape de concentration des phospholipides est réalisée sur une membrane céramique, couplée à un système d'uniformisation de la pression transmembranaire.

15. Procédé selon la revendication précédente, **caractérisé en ce que** la taille moyenne des pores est comprise entre 0,1 et 0,2 micromètres inclus, et **en ce que** la pression transmembranaire est maintenue à 0,6 Bar.

16. Procédé selon l'une quelconque des revendications 14 et 15, **caractérisée en ce qu'**une diafiltration continue est appliquée lorsque le facteur de concentration volumique est compris entre 1,2 et 4, et de préférence **en ce que** l'étape de concentration est réalisée jusqu'à l'obtention d'un facteur de concentration volumique de la composition laitière de préférence entre 2,5 et 5.

## Patentansprüche

1. Verfahren zur Herstellung eines Konzentrats von Phospholipiden, die aus einer mindestens Caseine und Phospholipide umfassenden flüssigen Milchzusammensetzung stammen, **dadurch gekennzeichnet, dass** es mindestens die folgenden Schritte umfasst:
- Leiten der Milchzusammensetzung über ein kationisches Harz in einer Ionenaustauschsäule,
- Aufkonzentrieren der Phospholipide der an Calcium verarmten Milchzusammensetzung durch Mikrofiltration mit kontrolliertem transmembranem Druckgradienten und
- Gewinnen des Retentats der Mikrofiltration und Erhalten eines Phospholipidkonzentrats.

2. Verfahren nach dem vorhergehenden Anspruch, **dadurch gekennzeichnet, dass** die Milchzusammensetzung Buttermilch und/oder Butterserum umfasst.

3. Verfahren nach einem der vorhergehenden Ansprüche, **dadurch gekennzeichnet, dass** das Verhältnis zwischen dem Konzentrationsfaktor der Proteine und dem Konzentrationsfaktor der Phospholipide kleiner als 1 ist, wobei jeder der Konzentrationsfaktoren dem Verhältnis zwischen dem Massenanteil, bezogen auf die Trockenmasse, im Retentat und dem Massenanteil, bezogen auf die Trockenmasse, in der Buttermilch vor der Mikrofiltration entspricht, und dadurch, dass der durch die Mikrofiltration bewirkte Proteinretentionsgrad kleiner als 70 % ist, und dadurch, dass das Verhältnis zwischen dem Konzentrationsfaktor der Proteine und dem Konzentrationsfaktor der Phospholipide kleiner als 0,9 ist.

4. Verfahren nach einem der vorhergehenden Ansprüche, **dadurch gekennzeichnet, dass** das kationische Harz der Ionenaustauschsäule ein schwaches kationisches Harz ist.

5. Verfahren nach einem der vorhergehenden Ansprüche, **dadurch gekennzeichnet, dass** im Verlauf des Phospholipidaufkonzentrationsschritts eine Diafiltration durchgeführt wird.

6. Verfahren nach dem vorhergehenden Anspruch, **dadurch gekennzeichnet, dass** die Diafiltration für einen Volumenkonzentrationsfaktor von weniger als 3 angewendet wird.

7. Verfahren nach einem der vorhergehenden Ansprüche, **dadurch gekennzeichnet, dass** der Phospholipidaufkonzentrationsschritt bei 50 °C durchgeführt wird.

8. Verfahren nach einem der vorhergehenden Ansprüche, **dadurch gekennzeichnet, dass** die bei der Mikrofiltration angewendete Tangentialgeschwindigkeit höher als 4 m/s ist.

9. Verfahren nach einem der vorhergehenden Ansprüche, **dadurch gekennzeichnet, dass** die mittlere Porengröße der Mikrofiltrationsmembran kleiner als 0,8 Mikrometer, bevorzugt kleiner als 0,5 Mikrometer ist.

10. Verfahren nach dem vorhergehenden Anspruch, **dadurch gekennzeichnet, dass** der transmembrane Druck zwischen 0,4 bar und 2 bar, bevorzugt zwischen 0,6 bar und 1 bar, einschließlich der Grenzen, liegt.

11. Verfahren nach einem der vorhergehenden Ansprüche, **dadurch gekennzeichnet, dass** der Phospholipidaufkonzentrationsschritt an einer Membran mit Permeabilitätsgradient durchgeführt wird.

12. Verfahren nach dem vorhergehenden Anspruch, **dadurch gekennzeichnet, dass** die Membran mit Permeabilitätsgradient eine mittlere Porengröße von 0,1 Mikrometer aufweist, und dadurch, dass der transmembrane Druck bei 1 bar gehalten wird.

13. Verfahren nach einem der Ansprüche 11 und 12, **dadurch gekennzeichnet, dass** eine kontinuierliche Diafiltration angewendet wird, wenn der Volumenkonzentrationsfaktor zwischen 1,2 und 3 beträgt, und vorzugsweise dadurch, dass der Aufkonzentrationsschritt durchgeführt wird, bis ein Volumenkonzentrationsfaktor der Milchzusammensetzung zwischen 2,5 und 5 erhalten wird.

14. Verfahren nach einem der Ansprüche 1 bis 10, **dadurch gekennzeichnet, dass** der Phospholipidaufkonzentrationsschritt an einer Keramikmembran durchgeführt wird, die mit einem System zur Vergleichmäßigung des transmembranen Drucks gekoppelt ist.

15. Verfahren nach dem vorhergehenden Anspruch, **dadurch gekennzeichnet, dass** die mittlere Porengröße zwischen 0,1 bis 0,2 Mikrometer, einschließlich der Grenzen, beträgt, und dadurch, dass der transmembrane Druck bei 0,6 bar gehalten wird.

16. Verfahren nach einem der Ansprüche 14 und 15, **dadurch gekennzeichnet, dass** eine kontinuierliche Diafiltration angewendet wird, wenn der Volumenkonzentrationsfaktor zwischen 1,2 und 4 beträgt, und vorzugsweise dadurch, dass der Aufkonzentrationsschritt durchgeführt wird, bis ein Volumenkonzentrationsfaktor der Milchzusammensetzung von vorzugsweise zwischen 2,5 und 5 erhalten wird.

## Claims

1. Method for producing a concentrate of phospholipids from a liquid dairy composition comprising at least caseins and phospholipids, **characterized in that** it comprises at least the steps of:
- passing the dairy composition over cationic resin in an ion exchange column,
- concentrating the phospholipids of the calcium-depleted dairy composition by controlled transmembrane pressure gradient microfiltration, and
- recovering the microfiltration retentate and obtaining a phospholipid concentrate.

2. Method according to the preceding claim, **characterized in that** the dairy composition comprises milk buttermilk and/or concentrated buttermilk.

3. Method according to either one of the preceding claims, **characterized in that** the ratio between the protein concentration factor and the phospholipid concentration factor is less than 1, each of said concentration factors corresponding to the ratio between the mass fraction of solids in the retentate and the mass fraction of solids in the buttermilk before microfiltration, and **in that** the protein retention rate induced by the microfiltration is less than 70%, and **in that** the ratio between the protein concentration factor and the phospholipid concentration factor is less than 0.9.

4. Method according to any one of the preceding claims, **characterized in that** the cationic resin of the ion exchange column is a weak cationic resin.

5. Method according to any one of the preceding claims, **characterized in that** a diafiltration is carried out during the phospholipid concentration step.

6. Method according to the preceding claim, **characterized in that** the diafiltration is applied for a volume concentration factor of less than 3.

7. Method according to any one of the preceding claims, **characterized in that** the phospholipid concentration step is carried out at 50°C.

8. Method according to any one of the preceding claims, **characterized in that** the tangential velocity applied to the microfiltration is greater than 4 m/s.

9. Method according to any one of the preceding claims, **characterized in that** the mean pore size of the microfiltration membrane is less than 0.8 micrometres, preferably less than 0.5 micrometres.

10. Method according to the preceding claim, **characterized in that** the transmembrane pressure is between 0.4 bar and 2 bar, preferably between 0.6 bar inclusive and 1 bar inclusive.

11. Method according to any one of the preceding claims, **characterized in that** the phospholipid concentration step is carried out on a permeability gradient membrane.

12. Method according to the preceding claim, **characterized in that** the permeability gradient membrane has a mean pore size of 0.1 micrometres, and **in that** the transmembrane pressure is maintained at 1 bar.

13. Method according to either one of Claims 11 and 12, **characterized in that** a continuous diafiltration is applied when the volume concentration factor is between 1.2 and 3, and preferably **in that** the concentration step is carried out until the volume concentration factor of the dairy composition is between 2.5 and 5.

14. Method according to any one of Claims 1 to 10, **characterized in that** the phospholipid concentration step is carried out on a ceramic membrane coupled to a system for harmonizing the transmembrane pressure.

15. Method according to the preceding claim, **characterized in that** the mean pore size is between 0.1 and 0.2 micrometres inclusive, and **in that** the transmembrane pressure is maintained at 0.6 bar.

16. Method according to either one of Claims 14 and 15, **characterized in that** a continuous diafiltration is applied when the volume concentration factor is between 1.2 and 4, and preferably **in that** the concentration step is carried out until the volume concentration factor of the dairy composition is preferably between 2.5 and 5.
